# EUROPEAN PATENT SPECIFICATION

(11) **EP 0 952 145 B1**
(45) Date of publication and mention of the grant of the patent: **05.10.2005**
(21) Application number: 98420070.9
(22) Date of filing: 20.04.1998
(51) Int. Cl.: C07C 255/66, C07C 253/30, C07D 213/77

(54) **Process for preparing pesticidal intermediates**
Verfahren zur Herstellung von pestiziden Zwischenprodukten
Procédé pour la préparation d'intermédiaires pesticides

(43) Date of publication of application: 27.10.1999
(73) Proprietor: BASF Agro B.V., Arnhem (NL), Wädenswil-Branch, 8820 Wädenswil/Au (CH)
(72) Inventor: Ancel, Jean Erick, 69230, Saint-Genis-Laval (FR)
(74) Representative: Vonend, Michael

(56) References cited:
- WO-A-97/32843
- DE-A- 3 612 940
- US-A- 4 824 960
- P. SCHMIDT ET AL.: "Heilmittelchemische Studien in der heterocyclischen Reihe" HELVETICA CHIMICA ACTA., vol. 41, 1958, pages 306-309, XP002079234

## Description

The invention relates to novel processes for preparing intermediates useful in the preparation of pesticides.

European Patent Publication Nos. 0295117 and 0234119 describe the preparation of pesticidally active phenylpyrazole compounds and of 5-amino-1-aryl-3-cyanopyrazole intermediate compounds used in their synthesis.

Various methods for preparing these compounds are known. The present invention seeks to provide improved or more economical methods for the preparation of pesticides and the intermediate compounds useful in preparing them.

Helv. Chim. Acta. Vol. 41, 1958, p306-309, describes on page 307 the oxidation of N-phenyl-N'-2-cyanoethyl-phenylhydrazine using (a) iron (III) sulphate and then (b) sodium hydroxide to produce 2-phenyl-3-aminopyrazole. The reaction scheme depicted in this reference shows azo-N-phenyl-N'-β-cyanoethyl as an intermediate product. That compound is depicted as being converted to the pyrazole via a postulated phenylhydrazono intermediate. The postulated route of the reaction is inferred from the failure of another possible intermediate (a pyrazoline) to dehydrogenate under similar conditions. The structure of the azo compounds in the postulated reaction scheme is only indicated as "probable" on the basis of analysis, UV and IR spectra.

US Patent No. 4,824,960 describes the preparation of 5-amino-1-arylpyrazole derivatives of general formula: 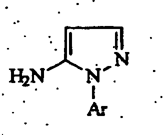 wherein Ar represents substituted phenyl or pyridyl, which can be used as intermediates in the preparation of compounds possessing herbicidal or pesticidal properties, by the reaction of arylhydrazines of formula:

Ar-NH-NH₂

wherein Ar is as hereinbefore defined, with acrylonitrile of formula:

NC-CH=CH₂

in a first stage in the presence of a diluent and optionally a catalyst to give the arylhydrazine compounds of the formula:

Ar-NH-NH-CH₂-CH₂-CN

wherein Ar is as hereinbefore defined, followed by oxidation and cyclisation in a second process stage.

However if it is desired to Perform an oxidation of the above 3-arylhydrazinopropionitriles (without cyclisation to the 5-amino-1-arylpyrazoles) in order to obtain 3-arylhydrazonopropionitriles, which may then be further processed to provide important 5-amino-1-aryl-3-cyanopyrazole compounds which are valuable intermediates in the preparation of pesticides, a different process must be employed

The present invention accordingly provides a process for the preparation of a compound of the formula (I): 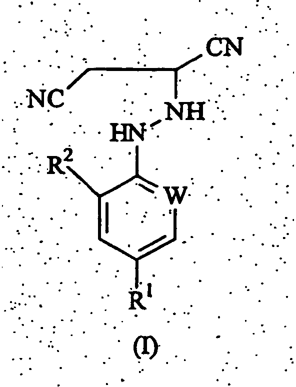 wherein W represents nitrogen or -CR³;
R¹ represents halogen, haloalkyl (preferably trifluoromethyl), haloalkoxy (preferably trifluoromethoxy), R⁴S(O)ₙ-, or -SF₅;
R² represents hydrogen or halogen (for example chlorine or bromine);
R³ represents halogen (for example chlorine or bromine);
R⁴ represents alkyl or haloalkyl; and
n represents 0,1 or 2; which process comprises the oxidation of a compound of formula (II): 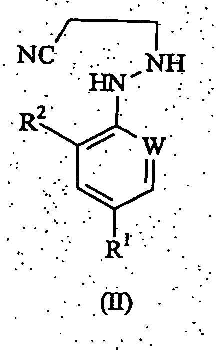 wherein R¹, R² and W are as hereinbefore defined; to prepare a compound of formula (III): 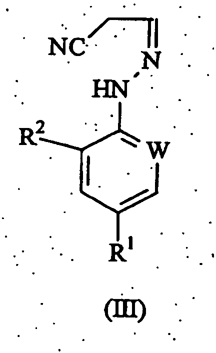 wherein R¹, R² and W are as hereinbefore defined; followed by the reaction of the compound of formula (III) with a source of hydrogen cyanide to prepare the compound of formula (I).

Unless otherwise specified in the present specification "alkyl" means straight- or branched- chain alkyl having from one to six carbon atoms (preferably one to three). Unless otherwise specified "haloalkyl" and "haloalkoxy" are straight- or branched- chain alkyl having from one to six carbon atoms (preferably one to three) substituted by one or more halogen atoms selected from fluorine, chlorine or bromine.

Suitable oxidants for the above reaction to form compounds of formula (III) include quinones such as benzoquinone, peroxides such as hydrogen peroxide, hypohalites such as sodium hypochlorite; or preferably a metal salt or oxide for example cupric chloride or mercuric oxide. The oxidation is generally conducted in a solvent. Solvents suitable for use include aromatic halogenated or non-halogenated hydrocarbons such as toluene or chlorobenzene, nitriles such as acetonitrile or amides such as N,N-dimethylformamide. The reaction temperature is generally from 20°C to 150°C, and preferably from 50°C to 100°C.

The molar ratio of oxidant to compound of formula (II) is generally from 0.01:1 to 5:1, preferably from1:1 to 3:1.

In formulae (I), (II) and (III) and in the formulae depicted hereinafter, preferred values of the symbols are as follows:-
R¹ represents haloalkyl (preferably trifluoromethyl), haloalkoxy (preferably trifluoromethoxy) or -SF₅;
W represents -CR³; and R³ represents halogen.

A most preferred compound of formula (III) is 3-(2,6-dichloro-4-trifluoromethylphenylhydrazono)propionitrile.

The process of the invention represents in certain aspects an improvement over the prior art.

Compounds of formula (III) may exist as a mixture of syn and anti isomers. Compounds of formula (I) may exist in the R- and S- forms.

The source of hydrogen cyanide which may be used in the reaction to prepare compounds of formula (I) from compounds of formula (III) may be hydrogen cyanide gas itself, when the reaction is optionally performed in the presence of a base, for example pyridine; but it is preferably prepared in situ (to avoid the direct use of hydrogen cyanide) from a metal cyanide salt (generally an alkali metal cyanide, for example sodium cyanide or potassium cyanide), in the presence of an acid. Suitable acids include organic acids such as aliphatic carboxylic acids for example acetic acid, or halogenated aliphatic carboxylic acids such as chloroacetic acid or trifluoroacetic acid, or sulphonic acids such as benzenesulphonic acid, 4-toluenesulphonic acid or methanesulphonic acid; or inorganic acids such as hydrochloric acid or sulphuric acid.

Alternative sources of hydrogen cyanide (which may be generated in situ) are trimethylsilylcyanide in water, or a mixture of trimethylsilylcyanide and a Lewis acid for example tin (IV) tetrachloride, in a solvent such as dichloromethane or tetrahydrofuran, at a temperature of from 20°C to 100°C, preferably from 30°C to 60°C. The reaction is preferably performed under increased pressure which increases the speed of the reaction.

The preparation of compounds of formula (I) from compounds of formula (III) may be effected in a polar or a non-polar solvent. Examples of polar solvents which may be used include water; alcohols such as methanol or ethanol; N,N-dimethylformamide; dimethylsulphoxide; or alkanoic acids such as acetic acid. Examples of non-polar solvents include hydrocarbons such as hexane, or ethers such as tetrahydrofuran or dioxane, or dialkyl ethers such as diethyl ether. When a metal cyanide salt is used in the presence of an acid, the prepared solvent is water or a mixture of water with a water miscible solvent. The reaction temperature is generally from 0°C to 100°C, preferably from 20°C to 50°C.

Most preferably the compound of formula (I) is 2-(2,6-dichloro-4-trifluoromethylphenylhydrazino)succinonitrile.

According to a further feature of the invention the compound of formula (II) used as starting material is prepared by the reaction of an arylhydrazine compound of formula (IV): 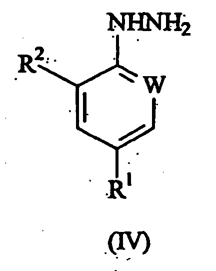 wherein R¹, R² and W are as hereinbefore defined; with acrylonitrile of formula (V):

NC-CH=CH₂ (V)

to give a compound of formula (II) as defined above.

Compounds of formula (IV) and (V) are known.

The compounds of formula (I) obtained by the process of the invention may be used in the preparation of pesticidally active 5-amino 1-aryl-3-cyanopyrazole derivatives of formula (VI) obtained from intermediate compounds of formula (V) and (VII) according to the following reaction scheme: 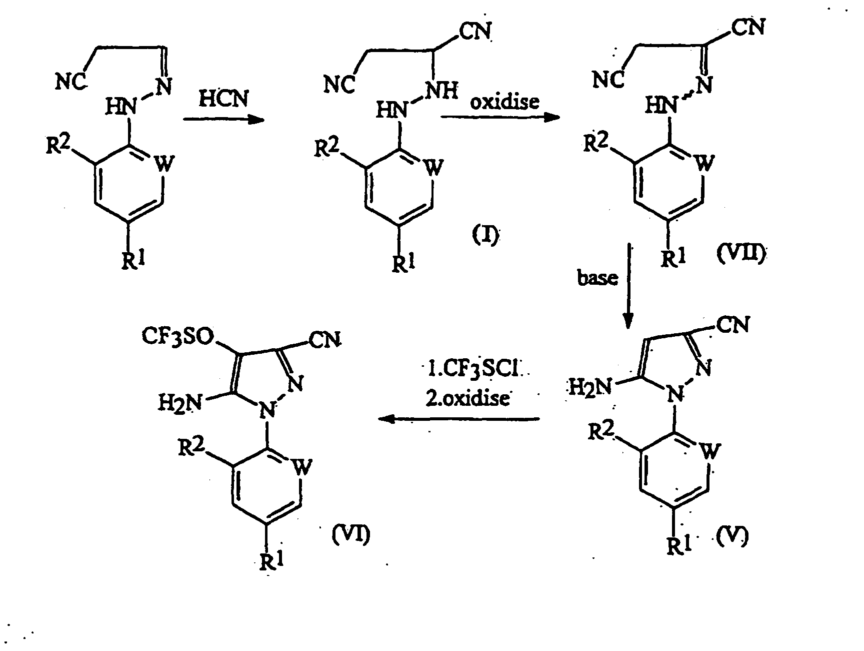 wherein the symbols used above are as hereinbefore defined.

The following non-limiting examples illustrate the invention. NMR spectra are recorded using deuterochloroform as solvent.

### Example 1

Cupric chloride (0.673g, 2.5 equivalents) was added in one portion to a solution of 3-(2,6-dichloro-4-trifluoromethylphenylhydrazino)propionitrile (0591g, 2mmol) in chlorobenzene, and the mixture heated at 65°C for 50 minutes. The reaction was judged to be complete and was cooled, washed (water), dried (magnesium sulphate), evaporated and separated by chromatography on silica gel to give 3-(2,6-dichloro-4-trifluoromethylphenylhydrazono)propionitrile,
NMR 3.37 (d, 2H), 7.03 (t,1H), 7.5 (s, 2H), 7.75 (s,1H) (35% yield), and 3-(2,6-dichloro-4-trifluoromethylphenylazo)propionitrile, NMR 3.0 (t,2H), 4.6 (t,2H), 7.6 (s,2H) (60% yield).

### Example 2

### Preparation of 2-(2,6-dichloro-4-trifluoromethylphenylhydrazino)succinonitrile

2-(2,6-Dichloro-4-trifluoromethylphenylhydrazono) propionitrile (0.296g, 1mmol), sodium cyanide (0.196g, equivalents), water (1ml) and acetic acid (5ml) were added successively to a tube, which was sealed and reacted at 20°C for 40 hours. The mixture was added to saturated sodium bicarbonate solution, extracted (dichloromethane), washed (water), dried (magnesium sulphate) and evaporated to give a mixture which contained 40% of the desired title compound, NMR 3.1 (m, 2H), 4.5 (m,1H), 5.89 (m, 1H), 6.94 (d,1H), 7.71 (s,2H), together with 60% of unchanged starting hydrazone.

### Reference Example

### i) Preparation of 2-(2,6-dichloro-4-trifluoromethylphenylhydrazono)succinonitrile

A mixture of 2-(2,6-dichloro-4-trifluoromethylphenylhydrazino) succinonitrile (0.323g) and cupric chloride (0.175g) was heated in chlorobenzene at 60°C for 6 hours. After filtration and evaporation the title compound and 5-amino-3-cyano-1-(2,6-dichloro-4-trifluoromethylphenyl)pyrazole were obtained as a 7:1 mixture Column chromatography on silica gel eluting with dichloromethane gave the pure title compound, obtained as a mixture of syn and anti isomers, NMR (anti isomer) 3.6 (s,2H), 7.57 (s,2H), 8.82 (s,1H, exchangeable with D₂O), NMR (syn isomer) 3.56 (s,2H), 7.59 (s,2H), 8.27 (s,1H, exchangeable with D₂O).

### ii) Preparation of 5-amino-3-cyano-1-(2,6-dichloro-4-trifluoromethylphenyl)pyrazole

Ammonia (20 microlitres of an 8% ammonia solution in water) was added to a mixture of the above 2-(2,6-dichloro-4-trifluoromethylphenylhydrazono) -succinonitrile (0.077g) in ethanol (1ml) and water (0.2ml) at 0 °C. After 10 minutes the mixture was extracted (dichloromethane) and evaporated to give 5-amino-3-cyano-1-(2,6-dichloro-4-trifluoromethylphenyl)pyrazole (0.076g, 97% yield). Purity 98% (by hplc).

## Claims

1. A process for the preparation of a compound of formula (I): 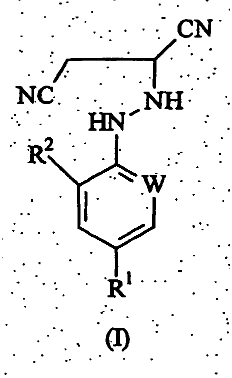 wherein W represents nitrogen or -CR³;
R¹ represents halogen, haloalkyl, haloalkoxy, R⁴S(O)ₙ₋ or -SF₅;
R² represents hydrogen or halogen;
R³ represents halogen;
R⁴ represents alkyl or haloalkyl; and
n represents 0,1 or 2; which process comprises the oxidation of a compound of formula (II): 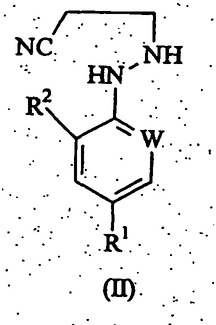 wherein R¹, R² and W are as hereinbefore defined; to prepare a compound of formula (III): 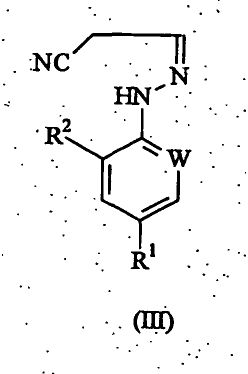 wherein R¹, R² and W are as hereinbefore defined; followed by the reaction of the compound of formula (in) with a source of hydrogen cyanide to prepare the compound of formula (I).

2. A process according to claim 1 in which the oxidant is a metal salt or oxide.

3. A process according to claim 1 or 2 in which the oxidation is conducted in a solvent.

4. A process according to any one of claims 1 to 3 in which the molar ratio of oxidant to compound of formula (II) is from 0.01:1 to 5:1.

5. A process according to claim 4 in which the ratio is from 1:1 to 3:1.

6. A process according to any one of the preceding claims, in which the compound of formula (II) is prepared by the reaction of a compound of formula (IV): 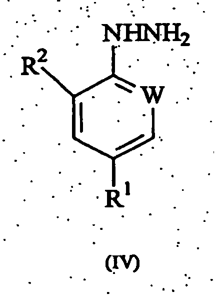 wherein R¹, R² and W are as defined in claim 1, with acrylonitrile of formula (V):
NC-CH=CH₂ (V)

7. A process according to any one of the preceding claims wherein:
R¹ represents haloalkyl, haloalkoxy or -SF₅;
W represents -CR³; and R³ represents halogen.

8. A process according to any one of the preceding claims wherein in the definition of R¹ haloalkyl is trifluoromethyl and haloalkoxy is trifluoromethoxy; and in the definitions of R² and R³ halogen is chlorine or bromide.

9. A process according to any one of the preceding claims wherein:
R¹ represents trifluoromethyl; W represents -CR³; and R² and R³ represent chlorine.

## Patentansprüche

1. Verfahren zur Herstellung einer Verbindung der Formel (I), 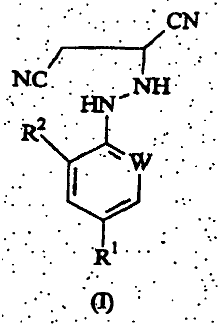 in der W Stickstoff oder -CR³ bedeutet,
R¹ Halogen, Halogenalkyl, Halogenalkoxy, R⁴S(O)ₙ- oder -SF5 bedeutet;
R² Wasserstoff oder Halogen bedeutet;
R³ Halogen bedeutet;
R⁴ Alkyl oder Halogenalkyl bedeutet; und
n 0, 1 oder 2 bedeutet,
**dadurch gekennzeichnet, daß** man eine Verbindung der Formel (II), 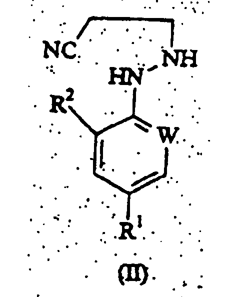 in der R¹, R² und W wie oben definiert sind,
zu einer Verbindung der Formel (III), 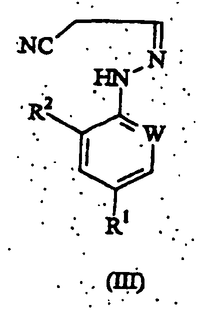 in der R¹, R² und W wie oben definiert sind, oxidiert und anschließend die Verbindung der Formel (III) mit einer Cyanwasserstoffquelle zur Verbindung der Formel (I) umsetzt.

2. Verfahren nach Anspruch 1, wobei es sich bei dem Oxidationsmittel um ein Metallsalz oder Oxid handelt.

3. Verfahren nach Anspruch 1 oder 2, wobei in einem Lösungsmittel oxidiert wird.

4. Verfahren nach einem der Ansprüche 1 bis 3, wobei das Molverhältnis Oxidationsmittel zur Verbindung der Formel (II) 0,01:1 bis 5:1 beträgt.

5. Verfahren nach Anspruch 4, wobei dieses Verhältnis 1:1 bis 3:1 beträgt.

6. Verfahren nach einem der vorhergehenden Ansprüche, in dem die Verbindung der Formel (II) **dadurch** hergestellt wird, daß man eine Verbindung der Formel (IV), 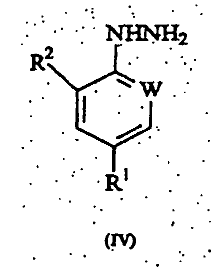 in der R¹, R² und W wie in Anspruch 1 definiert sind,
mit Acrylnitril der Formel (V)
NC-CH=CH₂ (V)
umsetzt.

7. Verfahren nach einem der vorhergehenden Ansprüche, wobei
R¹ Halogenalkyl, Halogenalkoxy oder -SF₅ bedeutet;
W -CR³ bedeutet; und R³ Halogen bedeutet.

8. Verfahren nach einem der vorhergehenden Ansprüche, wobei in der Definition von R¹ Halogenalkyl Trifluormethyl und Halogenalkoxy Trifluormethoxy bedeuten und in den Definitionen von R² und R³ Halogen Chlor oder Brom bedeutet.

9. Verfahren nach einem der vorhergehenden Ansprüche, wobei
R¹ Trifluormethyl bedeutet; W -CR³ bedeutet; und R² und R³ Chlor bedeuten.

## Revendications

1. Procédé de préparation d'un composé de formule (I) : 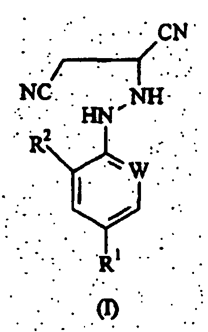 dans laquelle W représente de l'azote ou -CR³,
R¹ représente de l'halogène ou un groupe halogénoalkyle, halogénoalcoxy, R⁴S(O)ₙ n ou -SF₅,
R² représente de l'hydrogène ou de l'halogène,
R³ représente de l'halogène,
R⁴ représente un groupe alkyle ou halogénoalkyle, et
n représente 0, 1 ou 2, lequel procédé comprend l'oxydation d'un composé de formule (II) : 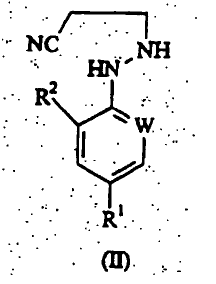
dans laquelle R¹, R² et W sont définis comme ci-dessus, pour préparer un composé de formule (III) : 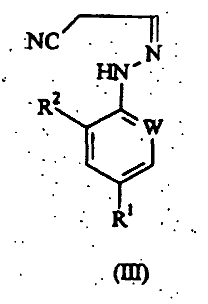 dans laquelle
R¹, R² et W sont définis comme ci-dessus, suivi par la réaction du composé de formule (III) avec une source de cyanure d'hydrogène pour préparer le composé de formule (I).

2. Procédé suivant la revendication 1, dans lequel l'oxydant est un sel ou oxyde métallique.

3. Procédé suivant la revendication 1 ou 2, dans lequel l'oxydation est effectuée dans un solvant.

4. Procédé suivant l'une quelconque des revendications 1 à 3, dans lequel le rapport molaire entre oxydant et composé de formule (II) est de 0,01/1 à 5/1.

5. Procédé suivant la revendication 4, dans lequel le rapport est de 1/1 à 3/1.

6. Procédé suivant l'une quelconque des revendications précédentes, dans lequel le composé de formule (II) est préparé par la réaction d'un composé de formule (IV) : 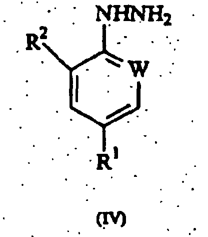 dans laquelle R¹, R² et W sont tels que définis dans la revendication 1, avec de l'acrylonitrile de formule (V) :
NC-CH=CH₂ (V)

7. Procédé suivant l'une quelconque des revendications précédentes, dans lequel
R¹ représente un groupe halogénoalkyle, halogénoalcoxy ou -SF₅,
W représente -CR³, et R³ représente de l'halogène.

8. Procédé suivant l'une quelconque des revendications précédentes, dans lequel, dans la définition de R¹, l'halogénoalkyle est du trifluorométhyle et l'halogénoalcoxy est du trifluorométhoxy et, dans les définitions de R² et R³, l'halogène est du chlore ou du brome.

9. Procédé suivant l'une quelconque des revendications précédentes, dans lequel
R¹ représente du trifluorométhyle, W représente -CR³, et R² et R³ représentent du chlore.
